# EUROPEAN PATENT SPECIFICATION

(11) **EP 4 079 265 B1**
(45) Date of publication and mention of the grant of the patent: **18.10.2023**
(21) Application number: 21169085.4
(22) Date of filing: 19.04.2021
(51) Int. Cl.: A61F 5/37

(54) **SHOULDER BRACE**
SCHULTERSTÜTZE
ÉPAULIERE

(43) Date of publication of application: 26.10.2022
(73) Proprietor: Roessingh Revalidatie Techniek B.V., 7522 AH Enschede (NL)
(72) Inventor: de Koning, John-John, 7545 XL Enschede (NL); van Vliet, Reinout Otto, 7524 BG Lonneker (NL)
(74) Representative: 't Jong, Bastiaan Jacob

(56) References cited:
- EP-A1- 3 235 474
- US-A1- 2010 069 802
- US-A1- 2017 281 384

## Description

The invention relates to a shoulder brace as defined in the claims. A shoulder brace for approximation of the humerus head into the shoulder joint of a person, which shoulder brace comprises:
- a shoulder pad adapted to be positioned over the top of a shoulder of a person, which shoulder pad has a dorsal end and a ventral end, and which shoulder pad configured to extend from the dorsal side over the top of the shoulder to the ventral side;
- an adjustable armpit belt, wherein both ends of the adjustable armpit belt are arranged to the shoulder pad, which adjustable armpit belt is adapted to run along the armpit opposite of the shoulder on top of which the shoulder pad is positioned;
- an upper arm sleeve adapted to be worn around the upper arm of a person and configured to enclose the upper arm and extend from the ventral side to the dorsal side of the upper arm;
- coupling means arranged with one end to the shoulder pad and with the other end to the upper arm sleeve, is known from US 5403268 and EP3235474.

Good positioning of the humerus head according to the joint surface will prevent or reduce the anterior and/or caudal (sub)luxation of humerus head.

This subluxation in most of the cases due to dysbalance of shoulder stabilizing muscles as a result of central neurological causes (like stroke), plexopathies, neuromuscular disorders or serious hypermobility syndromes (like Ehlers Danlos Syndrome). Traction of the shoulder structures caused by the weight and movements of the arm causes pain by local elongation of the joint capsule, microdamage of the glenohumeral joint or overload of residual stabilizing rotator cuff muscles because of overcompensation

A commonly known manner of relieving the shoulder is by use of a sling. The sling is wrapped around the forearm and around the neck of the person. As a result the weight of the arm is borne by the neck and the arm is immobilized against the body. The neck is however not suited for carrying such a weight and after a day wearing a sling, the muscles in the neck will start to ache. Also, the fact that one of the arms cannot be properly used, as it is immobilized, will result in overloading other parts of the body, such as the other arm and the back.

Improved slings are known in the prior art, in which the sling does not run over the neck, but runs over the shoulder. This relieves the neck, but the forearm is still immobilized by the sling being wrapped around it.

Another shoulder brace is known from US 5403268 and EP3235474.

Such a shoulder brace has a shoulder pad held in position by an adjustable arm pit belt. An upper arm sleeve is furthermore provided and mounted by one or more straps to the shoulder pad.

Such a shoulder brace allows for more freedom of movement compared to for example a sling. The shoulder brace provides support when the upper arm is hanging downwards, but as soon as the upper arm is lifted, the distance between the mounting points on the upper arm sleeve and the shoulder pad changes, such that the support for the shoulder is changed.

So, the known shoulder braces either provide insufficient support, such that the shoulder is not relieved sufficiently and might cause blood circulation problems, or the known shoulder braces provide sufficient support, while immobilizing the full arm.

It is an object of the invention to reduce or even remove the above mentioned disadvantages.

This object is achieved according to the invention with a shoulder brace according to the preamble, which is characterized in that the coupling means comprise:
- at least one flexible tube running over the shoulder pad from the dorsal end to the ventral end;
- at least one wire running through the at least one flexible tube, wherein both ends of the at least one wire are arranged to the upper arm sleeve on opposite sides relative to the axis of the upper arm sleeve.

When the shoulder brace is worn by a user, the at least one flexible tube thus extends in a plane substantially parallel to the sagittal plane.

The at least one wire is able to slide through the at least one flexible tube. So, when the person wearing the shoulder brace moves the upper arm back and forth parallel to the sagittal plane, the increased distance between a mounting point on one side on the upper arm sleeve and the shoulder pad is compensated, as the wire can slide though the flexible tube, by the decreased distance of a mounting on the opposite side of the upper arm sleeve.

In a preferred embodiment of the shoulder brace according to the invention at least one section of the at least one flexible tube is fixed to the shoulder pad and wherein both ends of the at least one flexible tube are arranged free.

If the upper arm is moved in the frontal plane, the distance between the shoulder pad and the mounting of the end of the wire will increase. By providing the flexible tube with free ends, these ends can bend towards the movement of the upper arm such that the distance between the shoulder pad and the mounting of the ends on the upper arm sleeve are maintained more or less equal.

In a preferred embodiment of the shoulder brace according to the invention the free ends of the at least one flexible tube are at least 5 cm long. This allows for sufficient flexibility to allow for movement of the upper arm in the frontal plane.

In yet a further preferred embodiment of the shoulder brace according to the invention at least two flexible tubes run parallel over the shoulder pad from the dorsal end to the ventral end and wherein wires run through each of the at least two flexible tubes and wherein both ends of each wire are arranged to the upper arm sleeve on opposite sides relative to the axis of the upper arm sleeve.

Having at least two flexible tubes with wires running there through provides a more stable support of the upper arm sleeve and thus for a more stable support of the shoulder.

In an even more preferred embodiment of the shoulder brace according to the invention the ends of both wires on one side of the upper arm sleeve are connected to each other and guided around a guide mounted on said one side of the upper arm sleeve.

By connecting the ends of both wires on onse side of the upper arm sleeve, a single wire is formed, which runs through a first tube along the guide and back through the second tube. This further improves the stable support of the shoulder.

In another embodiment of the shoulder brace according to the invention the flexibility of the free ends of the two flexible tubes differ between the two flexible tubes. A more stiff tube will compensate less the distance change when the upper arm is moved in the frontal plane.

In a further preferred embodiment of the shoulder brace according to the invention at least one end of the at least one wire is linked to a lockable spool rotationally arranged on the upper arm sleeve.

The spool allows for a quick adjustment of the length of the wires, such that the shoulder brace can be fitted on a specific person.

Preferably, the shoulder brace further comprises a tightening mechanism attached to the upper arm sleeve and coupled to the spool, the tightening mechanism having a spring for winding a first length of cable around the spool and a manual control for manually winding a second length of cable around the spool to adjust the length of the wire.

The spring allows for any play on the wire, which contributes to the wear comfort of the shoulder brace according to the invention.

These and other features of the invention will be elucidated in conjunction with the accompanying drawings.
Figure 1 shows a front view of a person wearing an embodiment of a shoulder brace according to the invention.
Figure 2 shows a back view of the person of figure 1.
Figure 3 shows a detailed view of the person of figure 1 with his upper arm lifted in the frontal plane.

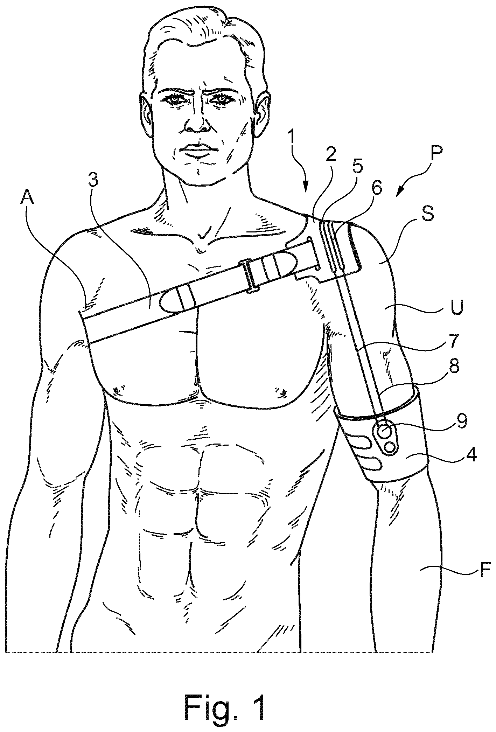

Figure 1 shows a person P wearing a shoulder brace 1 according to the invention. The shoulder brace 1 has a shoulder pad 2 arranged over the shoulder S of the person P. An adjustable arm pit belt 3, which is attached to the shoulder pad 2 and runs under the arm pit A, keeps the shoulder pad 2 in position. Furthermore, an upper arm sleeve 4 is arranged around the upper arm U near the elbow where the forearm F starts.

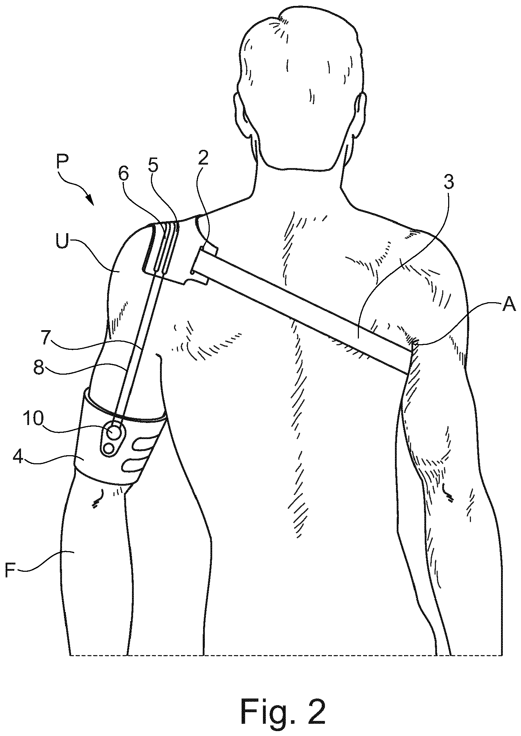

The shoulder pad 2 is provided with two flexible tubes 5, 6 which run over the shoulder pad 2 from the dorsal end (see figure 2) to the ventral end (see figure 1). Two wires 7, 8 run through the flexible tubes 5, 6 respectively and are wound with one end on a spool 9 and are connected with the other ends to each other and are guided along a guide 10 (see figure 2).

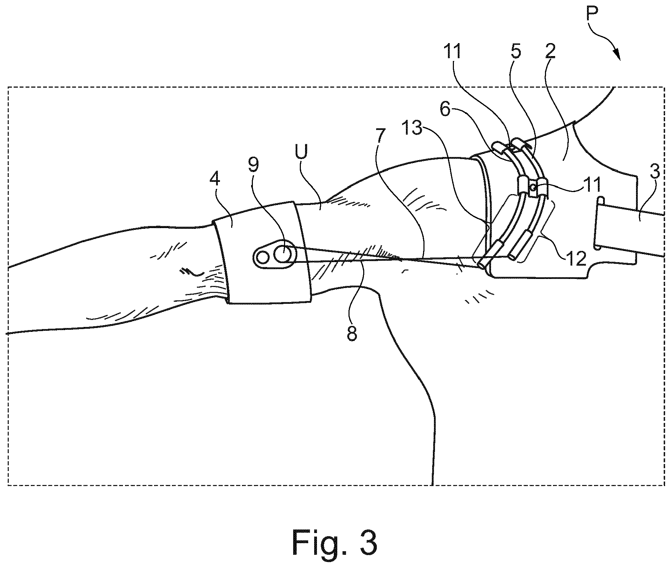

Figure 3 shows a more detailed view of the person P of figure 1 with his upper arm U lifted up in the frontal plane.

The flexible tubes 5, 6 are mounted to the shoulder pad 2 with mounts 11, leaving each flexible tube 5, 6 with a free end 12, 13 respectively.

As shown, the free ends 12, 13 of the flexible tubes 5, 6 will bend when the person P lifts his upper arm U in the frontal plane and compensate for the change in distance between the spool 9 and the ends of the flexible tubes 5, 6 if they were fixed to the shoulder pad 2 and would not have been free.

## Claims

1. Shoulder brace (1) for approximation of the humerus head into the shoulder joint of a person (P), which shoulder brace (1) comprises:
- a shoulder pad (2) adapted to be positioned over the top of a shoulder (S) of a person (P), which shoulder pad (2) has a dorsal end and a ventral end, and which shoulder pad (2) configured to extend from the dorsal side over the top of the shoulder (S) to the ventral side;
- an adjustable armpit belt (3), wherein both ends of the adjustable armpit belt (3) are arranged to the shoulder pad (2), which adjustable armpit belt (3) is adapted to run along the armpit (A) opposite of the shoulder (S) on top of which the shoulder pad (2) is positioned;
- an upper arm sleeve (4) adapted to be worn around the upper arm (U) of a person (P) and configured to enclose the upper arm (U) and extend from the ventral side to the dorsal side of the upper arm (U);
- coupling means arranged with one end to the shoulder pad (2) and with the other end to the upper arm sleeve (4),
**characterized in that**
the coupling means comprise:
- at least one flexible tube (5, 6) running over the shoulder pad (2) from the dorsal end to the ventral end;
- at least one wire (7, 8) running through the at least one flexible tube (5, 6), wherein both ends of the at least one wire (7, 8) are arranged to the upper arm sleeve (4) on opposite sides relative to the axis of the upper arm sleeve (4) .

2. Shoulder brace (1) according to claim 1, wherein at least one section of the at least one flexible tube (5, 6) is fixed to the shoulder pad (2) and wherein both ends (12, 13) of the at least one flexible tube (5, 6) are arranged free.

3. Shoulder brace (1) according to claim 2, wherein the free ends (12, 13) of the at least one flexible tube (5, 6) are at least 5 cm long.

4. Shoulder brace (1) according to any of the preceding claims, wherein at least two flexible tubes (5, 6) run parallel over the shoulder pad (2) from the dorsal end to the ventral end and wherein wires (7, 8) run through each of the at least two flexible tubes (5, 6) and wherein both ends of each wire (7, 8) are arranged to the upper arm sleeve (4) on opposite sides relative to the axis of the upper arm sleeve (4) .

5. Shoulder brace (1) according to claim 4, wherein the ends of both wires (7, 8) on one side of the upper arm sleeve (4) are connected to each other and guided around a guide (10) mounted on said one side of the upper arm sleeve (4) .

6. Shoulder brace (1) according to claim 2 and 4 or according to claim 2 and 5, wherein the flexibility of the free ends (12, 13) of the two flexible tubes (5, 6) differ between the two flexible tubes (5, 6).

7. Shoulder brace (1) according to any of the preceding claims, wherein at least one end of the at least one wire (7, 8) is linked to a lockable spool (9) rotationally arranged on the upper arm sleeve (4).

8. Shoulder brace (1) according to claim 7, further comprising a tightening mechanism attached to the upper arm sleeve (4) and coupled to the spool (9), the tightening mechanism having a spring for winding a first length of cable (7, 8) around the spool (9) and a manual control for manually winding a second length of cable (7, 8) around the spool to adjust the length of the wire (7, 8).

## Patentansprüche

1. Schulterstütze (1) zur Annäherung des Oberarmkopfes an das Schultergelenk einer Person (P), wobei die Schulterstütze (1) umfasst:
- ein Schulterpolster (2), das dazu ausgelegt ist, über der Oberseite einer Schulter (S) einer Person (P) positioniert zu werden, wobei das Schulterpolster (2) ein dorsales Ende und ein ventrales Ende aufweist und welches Schulterpolster (2) so konfiguriert ist, dass es sich von der dorsalen Seite über die Oberseite der Schulter (S) zur ventralen Seite erstreckt;
- einen verstellbaren Achselgürtel (3), wobei beide Enden des verstellbaren Achselgürtels (3) am Schulterpolster (2) angeordnet sind, wobei der verstellbare Achselgürtel (3) dazu ausgelegt ist, entlang der Achselhöhle (A) gegenüberliegend der Schulter (S) zu verlaufen, auf der das Schulterpolster (2) positioniert ist;
- eine Oberarmmanschette (4), die dazu ausgelegt ist, um den Oberarm (U) einer Person (P) getragen zu werden, und so konfiguriert ist, dass sie den Oberarm (U) umschließt und sich von der ventralen Seite zur dorsalen Seite des Oberarms (U) erstreckt;
- Kopplungsmittel, die mit einem Ende am Schulterpolster (2) und mit dem anderen Ende an der Oberarmmanschette (4) angeordnet sind,
**dadurch gekennzeichnet, dass**
die Kopplungsmittel umfassen:
- mindestens einen flexiblen Schlauch (5, 6), der über das Schulterpolster (2) vom dorsalen Ende zum ventralen Ende verläuft;
- mindestens ein Draht (7, 8), der durch den mindestens einen flexiblen Schlauch (5, 6) verläuft, wobei beide Enden des mindestens einen Drahtes (7, 8) an der Oberarmmanschette (4) auf gegenüberliegenden Seiten relativ zur Achse der Oberarmmanschette (4) angeordnet sind.

2. Schulterstütze (1) nach Anspruch 1, wobei mindestens ein Abschnitt des mindestens einen flexiblen Schlauchs (5, 6) am Schulterpolster (2) befestigt ist und wobei beide Enden (12, 13) des mindestens einen flexiblen Schlauchs (5, 6) frei angeordnet sind.

3. Schulterstütze (1) nach Anspruch 2, wobei die freien Enden (12, 13) des mindestens einen flexiblen Schlauchs (5, 6) mindestens 5 cm lang sind.

4. Schulterstütze (1) nach einem der vorstehenden Ansprüche, wobei mindestens zwei flexible Schläuche (5, 6) parallel über das Schulterpolster (2) vom dorsalen Ende zum ventralen Ende verlaufen und wobei Drähte (7, 8) durch jeden der mindestens zwei flexiblen Schläuche (5, 6) verlaufen und wobei beide Enden jedes Drahtes (7, 8) auf gegenüberliegenden Seiten relativ zur Achse der Oberarmmanschette (4) an der Oberarmmanschette (4) angeordnet sind.

5. Schulterstütze (1) nach Anspruch 4, wobei die Enden beider Drähte (7, 8) auf einer Seite der Oberarmmanschette (4) miteinander verbunden sind und um eine auf dieser einen Seite der Oberarmmanschette (4) angebrachte Führung (10) geführt sind.

6. Schulterstütze (1) nach Anspruch 2 und 4 oder nach Anspruch 2 und 5, wobei die Flexibilität der freien Enden (12, 13) der beiden flexiblen Schläuche (5, 6) zwischen den beiden flexiblen Schläuchen (5, 6) unterschiedlich ist.

7. Schulterstütze (1) nach einem der vorstehenden Ansprüche, wobei mindestens ein Ende des mindestens einen Drahtes (7, 8) mit einer arretierbaren Spule (9) verbunden ist, die drehbar an der Oberarmmanschette (4) angeordnet ist.

8. Schulterstütze (1) nach Anspruch 7, weiter umfassend einen Spannmechanismus, der an der Oberarmmanschette (4) befestigt und mit der Spule (9) gekoppelt ist, wobei der Spannmechanismus eine Feder zum Aufwickeln einer ersten Kabellänge (7, 8) um die Spule (9) und eine manuelle Steuerung zum manuellen Aufwickeln einer zweiten Kabellänge (7, 8) um die Spule aufweist, um die Länge des Drahtes (7, 8) anzupassen.

## Revendications

1. Orthèse d'épaule (1) pour rapprocher la tête de l'humérus dans l'articulation de l'épaule d'une personne (P), laquelle orthèse d'épaule (1) comprend :
- une épaulière (2) conçue pour être positionnée sur le dessus d'une épaule (S) d'une personne (P), laquelle épaulière (2) présente une extrémité dorsale et une extrémité ventrale et laquelle épaulière (2) est configurée pour s'étendre du côté dorsal sur le dessus de l'épaule (S) au côté ventral ;
- une sangle réglable sous l'aisselle (3), dans laquelle les deux extrémités de la sangle réglable sous l'aisselle (3) sont agencées sur l'épaulière (2), laquelle sangle réglable sous l'aisselle (3) est conçue pour s'étendre le long de l'aisselle (A) opposée de l'épaule (S) sur laquelle l'épaulière (2) est positionnée ;
- un manchon de bras (4) conçu pour être porté autour du bras (U) d'une personne (P) et configuré pour entourer le bras (U) et s'étendre du côté ventral au côté dorsal du bras (U) ;
- des moyens de couplage agencés avec une extrémité à l'épaulière (2) et avec l'autre extrémité au manchon de bras (4),
**caractérisée en ce que**
les moyens de couplage comprennent :
- au moins un tube flexible (5, 6) passant sur l'épaulette (2) de l'extrémité dorsale à l'extrémité ventrale ;
- au moins un fil (7, 8) s'étendant à travers le au moins un tube flexible (5, 6), dans laquelle les deux extrémités du au moins un fil (7, 8) sont agencées sur le manchon de bras (4) sur des côtés opposés par rapport à l'axe du manchon de bras (4).

2. Orthèse d'épaule (1) selon la revendication 1, dans laquelle au moins une section du au moins un tube flexible (5, 6) est fixée à l'épaulière (2) et dans laquelle les deux extrémités (12, 13) du au moins un tube flexible (5, 6) sont agencées librement.

3. Orthèse d'épaule (1) selon la revendication 2, dans laquelle les extrémités libres (12, 13) du au moins un tube flexible (5, 6) présentent une longueur d'au moins 5 cm.

4. Orthèse d'épaule (1) selon l'une quelconque des revendications précédentes, dans laquelle au moins deux tubes flexibles (5, 6) s'étendent parallèlement sur l'épaulière (2) de l'extrémité dorsale à l'extrémité ventrale et dans laquelle des fils (7, 8) s'étendent à travers chacun des au moins deux tubes flexibles (5, 6) et dans laquelle les deux extrémités de chaque fil (7, 8) sont agencées sur le manchon de bras (4) sur des côtés opposés par rapport à l'axe du manchon de bras (4).

5. Orthèse d'épaule (1) selon la revendication 4, dans laquelle les extrémités des deux fils (7, 8) sur un côté du manchon de bras (4) sont reliées l'une à l'autre et guidées autour d'un guide (10) monté sur ledit un côté du manchon de bras (4).

6. Orthèse d'épaule (1) selon les revendications 2 et 4 ou selon les revendications 2 et 5, dans laquelle la flexibilité des extrémités libres (12, 13) des deux tubes flexibles (5, 6) varie entre les deux tubes flexibles (5, 6).

7. Orthèse d'épaule (1) selon l'une quelconque des revendications précédentes, dans laquelle au moins une extrémité du au moins un fil (7, 8) est reliée à un enrouleur verrouillable (9) agencé en rotation sur le manchon de bras (4).

8. Orthèse d'épaule (1) selon la revendication 7, comprenant en outre un mécanisme de serrage fixé au manchon de bras (4) et couplé à l'enrouleur (9), le mécanisme de serrage présentant un ressort pour enrouler une première longueur du câble (7, 8) autour de l'enrouleur (9) et une commande manuelle pour enrouler manuellement une seconde longueur du câble (7, 8) autour de l'enrouleur pour ajuster la longueur du fil (7, 8).
